# EUROPEAN PATENT SPECIFICATION

(11) **EP 3 884 280 B1**
(45) Date of publication and mention of the grant of the patent: **17.01.2024**
(21) Application number: 19809018.5
(22) Date of filing: 19.11.2019
(51) Int. Cl.: G01N 33/68

(54) **METHODS AND KITS FOR DETECTING LIVER DYSFUNCTION IN A SUBJECT**
VERFAHREN UND KITS ZUM NACHWEIS VON LEBERFUNKTIONSSTÖRUNGEN BEI EINEM PROBANDEN
PROCÉDÉS ET KITS PERMETTANT DE DIAGNOSTIQUER UN DYSFONCTIONNEMENT DU FOIE CHEZ UN SUJET

(30) Priority: 20.11.2018 EP 18306528
(43) Date of publication of application: 29.09.2021
(73) Proprietor: Institut National de la Santé et de la Recherche Médicale (INSERM), 75013 Paris (FR); Centre Hospitalier Régional Universitaire de Limoges, 87000 Limoges (FR); Université de Limoges, 87000 Limoges (FR)
(72) Inventor: EL BALKHI, Souleiman, 87042 Limoges (FR); SAINT MARCOUX, Franck, 87042 Limoges (FR); WOILLARD, Jean-Baptiste, 87042 Limoges (FR)
(74) Representative: Inserm Transfert
(86) International application number: PCT/EP2019/081801
(87) International publication number: WO 2020/104458

(56) References cited:
- WO-A2-2013/024100
- KLAMMT SEBASTIAN ET AL: "Albumin-binding function is reduced in patients with decompensated cirrhosis and correlates inversely with severity of liver disease assessed by model for end-stage liver disease", EUROPEAN JOURNAL OF GASTROENTEROLOGY AND HEPATOLOGY, LIPPINCOTT WILLIAMS AND WILKINS, UK , vol. 19, no. 3 1 January 2007 (2007-01-01), pages 257-263, XP009505681, ISSN: 0954-691X, DOI: 10.1097/MEG.0B013E3280101F7D Retrieved from the Internet: URL:https://epo.summon.serialssolutions.co m/2.0.0/link/0/eLvHCXMwtV1Lb9NAEF41qoSQEOI lKA9pDtzQCj82tvfYViUVkAuEA6doba_Boo0rx1VVf ha_kJl9-BEqRA9crMTZbBLNl_1md76ZYex1rDJVhFn FZaAVF5mc8yzKc06l0QodKvTJKTn567FYLdJPC_Fxb -Zb-g33_qul8R7amjJnb2HtflK8gY_R5nhFq-P1n-x uztzrDe14Tb4KEZcVNFKAoLykeP9QT9Ult5WapOW4o 1XkgBZ1235
- OETTL KARL ET AL: "Oxidative albumin damage in chronic liver failure: Relation to albumin binding capacity, liver dysfunction and survival", JOURNAL OF HEPATOLOGY, vol. 59, no. 5, 25 June 2013 (2013-06-25), pages 978-983, XP028755480, ISSN: 0168-8278, DOI: 10.1016/J.JHEP.2013.06.013
- PENGLEI GE ET AL: "Reduction in albumin binding function following liver resection in patients with and without cirrhosis", TRANSLATIONAL CANCER RESEARCH, vol. 5, no. 6, 1 December 2016 (2016-12-01), pages 756-764, XP055576518, ISSN: 2218-676X, DOI: 10.21037/tcr.2016.12.13
- NALDI MARINA ET AL: "Structural and functional integrity of human serum albumin: Analytical approaches and clinical relevance in patients with liver cirrhosis", JOURNAL OF PHARMACEUTICAL AND BIOMEDICAL ANALYSIS, vol. 144, 18 April 2017 (2017-04-18), pages 138-153, XP085152051, ISSN: 0731-7085, DOI: 10.1016/J.JPBA.2017.04.023
- DATABASE MEDLINE [Online] US NATIONAL LIBRARY OF MEDICINE (NLM), BETHESDA, MD, US; March 1989 (1989-03), PAGLIACCI M C ET AL: "Thyroid function tests in acute viral hepatitis: relative reduction in serum thyroxine levels due to T4-TBG binding inhibitors in patients with severe liver cell necrosis.", Database accession no. NLM2498420 & PAGLIACCI M C ET AL: "Thyroid function tests in acute viral hepatitis: relative reduction in serum thyroxine levels due to T4-TBG binding inhibitors in patients with severe liver cell necrosis.", JOURNAL OF ENDOCRINOLOGICAL INVESTIGATION MAR 1989, vol. 12, no. 3, March 1989 (1989-03), pages 149-153, ISSN: 0391-4097

## Description

### FIELD:

The present disclosure is in the field of medicine.

### BACKGROUND:

Most chronic liver diseases are notoriously asymptomatic, until cirrhosis with clinical decompensation occurs [1, 2]. Prevention of cirrhosis and the use of early diagnosis strategies, before and once it develops, are vital to maintain patients in a symptom-free state and to delay decompensation, and thus improve the outcome. This is particularly critical in liver transplanted patients. In early cirrhosis, conventional imaging and laboratory tests, often combined in scores, can lead to false-negative diagnosis [2]. Despite their lack of sensitivity and specificity, these tests are routinely used to explore the integrity of hepatocytes (aspartate transaminase and alanine transaminase), as well as the biliary (alkaline phosphatase and 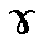 -glutamyltransferase) and synthesis (ammonia, prothrombin time and albumin) functions.

Since Human serum albumin (HSA) is exclusively synthesized and matured in the liver, not only its quantity (60% of all blood proteins normally) but also its quality may reflect liver dysfunction. Indeed, albumin exhibits a peculiar 3D structure, with multiple binding sites for multiple endogenous and exogenous ligands (it acts as the primary scavenger in blood).

Albumin undergoes several post-translational modifications in hepatocytes, including: acetylation, cysteinylation, homocysteinylation, glutathionylation, glycosylation, glycation, nitrosylation, nitration, phosphorylation and oxidation. The clinical relevance of some of these modifications has been recently investigated in advanced liver diseases (1-4)[1-4]. Such modifications in HSA structure translate in modifications of its conformation and binding properties [5]. This aspect has been exploited by Bar-Or et al. in cardiac ischemia, who proposed the albumin cobalt binding test (ACB) also known as the Ischemia Modified Albumin test (IMA) [6]. The IMA test is based on the fact that cardiac ischemia is associated with modifications in the structure of albumin and, thus, in the capacity of a specific binding site to bind cobalt. Since the approval of the IMA as a biomarker of cardiac ischemia by the FDA (Regulation number: 862.1215; http://www.accessdata.fda.gov), this test has also been investigated in liver diseases showing correlation with the severity of cirrhosis [7]. Briefly, the IMA test is performed by adding CoCh and dithiothreitol to serum, followed by a colorimetric measurement of the (free-Co)-dithiothreitol complex at 470 nm. However, there still a need for additional biomarker of liver dysfunction.

WO2013/024100 discloses methods for the diagnosis and the prevention or treatment of medical conditions associated with oxidative stress, in particular liver diseases, the methods being based on the determination and quantification of the fraction of albumin that is irreversibly oxidized at cysteine 34.

Ge et al. disclose that albumin binding function decreases following liver resection in patients with and without cirrhosis (Ge, Penglei et al. "Reduction in albumin binding function following liver resection in patients with and without cirrhosis". Transl Cancer Res 2016;5(6):756-764).

### SUMMARY:

The present invention is defined by the claims. In particular, the present invention relates to a method for determining whether a subject suffers from a liver dysfunction comprising measuring the binding capacities of gold (Au), copper (Cu), cadmium (cd), L-thyroxine and dansylsarcosine to serum albumin wherein said measured binding capacities indicate whether the subject suffers from a liver dysfunction. The following detailed description, figures and example do not fall under the scope of the present invention and are present for understanding and illustration purposes only.

### DETAILED DESCRIPTION:

The first hypothesis is that all the principal HSA modifications, due to a diversity of liver diseases, can be indirectly revealed by investigating the binding capacity for different ligands. It was reported that each of the following ligands has a specific binding site on HSA: (i) gold (Au) binds preferentially to Cys34; (ii) copper (Cu) to the N-terminal binding site, (iii) cadmium (Cd) to the multi-metal binding site, (iv) L-thyroxine has 4 specific binding sites (Tr1 - Tr4), and (v) dansylsarcosine was reported to bind to drug site 3 or to the diazepam-binding site [8]. The second hypothesis is that modifications of the HSA conformation and binding properties appear at early stages of liver cell injuries, since HSA is exclusively synthesized and matured in hepatocytes.

Therefore, we believe that the most frequent HSA structural modifications can be detected by measuring the free (unbound) ligands after spiking patient serum with solutions containing the abovementioned ligands. This is possible since Cu, Au and Cd cover the principal HSA binding sites, while dansylsarcosine and L-thyroxine could reflect its conformational modifications since their binding sites are located in the cavities of the protein. Thereafter, by revealing HSA modifications, liver dysfunction may be detected earlier than with conventional imaging or laboratory tests. Interestingly, all the cited ligands can be directly measured using a single method such as inductively coupled plasma mass spectrometry (ICP-MS) or inductively coupled plasma optical emission spectrometry (ICP-OES).

Based on these premises, we present here the serum enhanced binding (SEB) test, a simple laboratory test of liver dysfunctions. The SEB test was developed by analyzing serum samples from patients with different liver diseases (diagnosed cirrhosis, patients with NASH without cirrhosis and liver transplant patients...). Animal experimentations were also conducted to explore the precocity of HSA modifications in the course of chronic liver dysfunction.

Accordingly, the present disclosure relates to a method for determining whether a subject suffers from a liver dysfunction comprising measuring the binding capacities of gold (Au), copper (Cu), cadmium (cd), L-thyroxine and dansylsarcosine to serum albumin wherein said measured binding capacities indicate whether the subject suffers from a liver dysfunction.

In particular, the binding capacities of gold (Au), copper (Cu), cadmium (cd), L-thyroxine and dansylsarcosine are compared with their predetermined reference values, and the detection of a difference between the binding capacities of gold (Au), copper (Cu), cadmium (cd), L-thyroxine and dansylsarcosine and the predetermined reference values indicates if the subject suffers from a liver dysfunction.

In particular, the disclosure relates to a method for determining whether a subject suffers from a liver dysfunction comprising i) determining the binding capacities of serum albumin to gold (Au), copper (Cu), cadmium (cd), L-thyroxine and dansylsarcosine, ii) comparing the binding capacities determined at step i) with their predetermined reference values, wherein detecting difference between the binding capacities determined at step i) and the predetermined reference values indicates whether the subject suffers from a liver dysfunction

As used herein, the term "subject" as used herein refers to any mammal organism. The term subject includes, but is not limited to, humans, nonhuman primates such as chimpanzees and other apes and monkey species; farm animals such as cattle, sheep, pigs, goats and horses; domestic mammals such as dogs and cats; laboratory animals including rodents such as mice, rats and guinea pigs, and the like. The term does not denote a particular age or sex. Thus, adult and newborn subjects, as well as fetuses, whether male or female, are intended to be covered.

As used herein, the term "liver dysfunction" "or "hepatic dysfunction" refers to a state (e.g. the severity of the disease) in which the liver function is decreased relative to a normal state. Hepatic dysfunction is characteristic of liver diseases. A number of acute or chronic pathological conditions leads to liver dysfunction. These include, but are not limited to liver abscess, liver cancer, either primary or metastatic, cirrhosis, such as cirrhosis caused by the alcohol consumption or primary biliary cirrhosis, amebic liver abscess, autoimmune hepatitis, biliary atresia, coccidioidomycosis disseminated, portal hypertension hepatic infections (such as hepatitis A virus, hepatitis B virus, hepatitis C virus, hepatitis D virus, or hepatitis E virus), hemochromatosis, hepatocellular carcinoma, pyogenic liver abscess, Reye's syndrome, sclerosing cholangitis, Wilson's disease, drug induced hepatotoxicity, or fulminant or acute liver failure. In particular, the liver is a non-alcoholic fatty liver disease. As used herein, the term "non-alcoholic fatty liver disease" has its general meaning in the art and is intended to refer to the spectrum of disorders resulting from an accumulation of fat in liver cells in individuals with no history of excessive alcohol consumption. In the mildest form, NAFLD refers to hepatic steatosis. The term NAFLD is also intended to encompass the more severe and advanced form non-alcoholic steatohepatitis (NASH), cirrhosis, hepatocellular carcinoma, and virus- induced (e.g., HIV, hepatitis) fatty liver disease. The term "NASH", as used herein, collectively refers to the state where the liver develops a hepatic disorder (e.g., inflammation, ballooning, fibrosis, cirrhosis, or cancer), or the state where the liver may induce such a pathological condition, and "NASH" is distinguished from "simple steatosis"; i.e., a condition in which fat is simply accumulated in the liver, and which does not progress to another hepatic-disorder-developing condition.

In particular, the method of diagnosing described herein is applied to a subject who presents symptoms of liver dysfunction without having undergone the routine screening to rule out all possible causes for liver dysfunction. The methods described herein can be part of the routine set of tests performed on a subject who presents symptoms of liver dysfunction such as jaundice, abdominal pain and swelling, swelling in the legs and ankles, itchy skin, dark urine color, pale stool color, bloody color stool, tar-colored stool, chronic fatigue, nausea or vomiting, loss of appetite, tendency to bruise easily... The method of the present disclosure can be carried out in addition of other diagnostic tools that include ultrasound evaluation (e.g. elastography), biopsy and/or quantification of at least one further biomarkers such as levels of blood AST, ALT, ALP, TTT, ZTT, total bilirubin, total protein, albumin, lactate dehydrogenase, choline esterase and the like.

As used herein, the term "serum albumin" has its general meaning in the art and refers to a globular protein that in humans is encoded by the *ALB* gene. Serum albumin is the most abundant plasma protein in mammals. Serum albumin is essential for maintaining the oncotic pressure needed for proper distribution of body fluids between intravascular compartments and body tissues. It also acts as a plasma carrier by non-specifically binding several hydrophobic steroid hormones and as a transport protein for hemin and fatty acids. Furthermore, serum albumin has a very long half-life of about 19 days, and its metabolism is well-known. Albumin has also been widely used as a protein stabilizer in commercial pharmaceuticals (Sangastino et al. (2012), Blood, 120(12) 2405-2411). An exemplary amino acid sequence for human serum albumin (HSA) is represented by SEQ ID NO:1 (UniProtKB/Swiss-Prot primary accession number P02768).

SEQ ID NO:1 >sp|P02768|ALBU_HUMAN Serum albumin OS=Homo sapiens
OX=9606 GN=ALB PE=1 SV=2












As used herein, the term "ligand" refers to any molecule that has a specific binding site on albumin. In particular, the ligand is selected from the group consisting of gold (Au), copper (Cu), cadmium (cd), L-thyroxine and dansylsarcosine.

As used herein, the term "binding capacity" refers to the amount of the ligand that the serum albumin can bind under equilibrium conditions if every available binding site on the protein is utilized.

Typically, the method of the present disclosure is carried out as follows. In a first step a serum sample obtained from the subject is prepared. As used herein, the term "serum sample" relates to a sample wherein a blood sample is tapped into a dry-glass, left to coagulate at room temperature, and after which they are centrifuged. Then the serum sample is exposed to a predetermined amount of the ligand for a time sufficient for allowing the serum albumin to bind to said ligand. In particular, the time of exposure can be varied for about 1, 5 or 10 seconds, or about 1, 2, 3, 5, 10, 20 or 30 minutes, or about 1, 2, 3 or 5 hours. In a third step, the amount of the free (unbound) ligand is then measured in the sample, wherein said measure indicates the binding capacity of the serum albumin. Optionally a ratio between the free amount and the concentration of the serum albumin is calculated, wherein said ratio indicates the binding capacity of the serum albumin. Optionally, the protein contained in the sample are separated from the sample before measuring the amount of the free ligand. Typically said separation may consist in a centrifugation.

Accordingly, 5 serum samples are prepared separately and each exposed to a particular amount of the corresponding ligand. In particular, 5 containers (e.g. tubes) containing an amount of the corresponding ligand are prepared. The sample serum is then added to the container and finally after separating the proteins contained in the sample typically by a centrifugation the amount of the free ligand is measured in the resting sample.

In particular, the binding capacity (e.g. the amount of the free ligand) is determined by mass spectrometry.

As used herein, the term "Child-Pugh score" refers to a system for assessing the prognosis, including the required strength of treatment and necessity of liver transplant, of chronic liver disease, primarily cirrhosis. It provides a forecast of the increasing severity of your liver disease and your expected survival rate. The Child-Pugh score is defined by different class: "Class A" (CA) for least severe liver disease, "Class B (CB) for moderately severe liver disease, "Class C" (CC) for most severe liver disease.

As used herein, the term "mass spectrometry" or "MS" refers to an analytical technique to identify compounds by their mass. MS refers to methods of filtering, detecting, and measuring ions based on their m/z. MS technology generally includes (1) ionizing the compounds to form charged species (e.g., ions); and (2) detecting the molecular weight of the ions and calculating their m/z. The compounds may be ionized and detected by any suitable means. A "mass spectrometer" generally includes an ionizer and an ion detector. In general, one or more molecules of interest are ionized, and the ions are subsequently introduced into a mass spectrographic instrument where, due to a combination of magnetic and electric fields, the ions follow a path in space that is dependent upon mass ("m") and charge ("z"). See, e.g., U.S. Pat. No. 6,204,500, entitled "Mass Spectrometry From Surfaces;" U.S. Pat. No. 6,107,623, entitled "Methods and Apparatus for Tandem Mass Spectrometry;" U.S. Pat. No. 6,268,144, entitled "DNA Diagnostics Based On Mass Spectrometry;" U.S. Pat. No. 6,124,137, entitled "Surface-Enhanced Photolabile Attachment And Release For Desorption And Detection Of Analytes;" Wright et al., Prostate Cancer and Prostatic Diseases 2:264-76 (1999); and Merchant and Weinberger, Electrophoresis 21:1164-67 (2000).

Typically the serum samples are processed to obtain preparations that are suitable for analysis by mass spectrometry. Such purification will usually include chromatography, such as liquid chromatography or capillary electrophoresis, and may also often involve an additional purification procedure that is performed prior to chromatography. Various procedures may be used for this purpose depending on the type of sample or the type of chromatography. Examples include filtration, centrifugation, combinations thereof and the like. The pH of the serum sample may then be adjusted. The sample may be purified with a filtration. The filtrate from this filtration can then be purified by liquid chromatography and subsequently subjected to mass spectrometry analysis. Various methods have been described involving the use of high performance liquid chromatography (HPLC) for sample clean-up prior to mass spectrometry analysis. See, e.g., Taylor et al., Therapeutic Drug Monitoring 22:608-12 (2000) (manual precipitation of blood samples, followed by manual C18 solid phase extraction, injection into an HPLC for chromatography on a C18 analytical column, and MS/MS analysis); and Salm et al., Clin. Therapeutics 22 Supl. B:B71-B85 (2000). Commercially available HPLC columns include, but are not limited to, polar, ion exchange (both cation and anion), hydrophobic interaction, phenyl, C-2, C-8, C-18, and polar coating on porous polymer columns. During chromatography, the separation of materials is effected by variables such as choice of eluent (also known as a "mobile phase"), choice of gradient elution and the gradient conditions, temperature, etc.

In particular, the ligands are ionized by any method known to the skilled artisan. Mass spectrometry is performed using a mass spectrometer, which includes an ion source for ionizing the fractionated sample and creating charged molecules for further analysis. Ionization sources used in various MS techniques include, but are not limited to, electron ionization, chemical ionization, electrospray ionization (ESI), photon ionization, atmospheric pressure chemical ionization (APCI), photoionization, atmospheric pressure photoionization (APPI), fast atom bombardment (FAB)/liquid secondary ionization (LSIMS), matrix assisted laser desorption ionization (MALDI), field ionization, field desorption, thermospray/plasmaspray ionization, surface enhanced laser desorption ionization (SELDI), inductively coupled plasma (ICP) and particle beam ionization. The skilled artisan will understand that the choice of ionization method may be determined based on the analyte to be measured, type of sample, the type of detector, the choice of positive versus negative mode, etc. After the sample has been ionized, the positively charged ions thereby created may be analyzed to determine m/z. Suitable analyzers for determining m/z include quadrupole analyzers, ion trap analyzers, and time-of-flight analyzers. The ions may be detected using one of several detection modes. For example, only selected ions may be detected using a selective ion monitoring mode (SIM), or alternatively, multiple ions may be detected using a scanning mode, e.g., multiple reaction monitoring (MRM) or selected reaction monitoring (SRM). One may enhance the resolution of the MS technique by employing "tandem mass spectrometry," or "MS/MS." In this technique, a precursor ion (also called a parent ion) generated from a molecule of interest can be filtered in an MS instrument, and the precursor ion subsequently fragmented to yield one or more fragment ions (also called daughter ions or product ions) that are then analyzed in a second MS procedure. By careful selection of precursor ions, only ions produced by certain analytes are passed to the fragmentation chamber, where collision with atoms of an inert gas produce the fragment ions. Because both the precursor and fragment ions are produced in a reproducible fashion under a given set of ionization/fragmentation conditions, the MS/MS technique may provide an extremely powerful analytical tool. For example, the combination of filtration/fragmentation may be used to eliminate interfering substances, and may be particularly useful in complex samples, such as biological samples. Additionally, recent advances in technology, such as matrix-assisted laser desorption ionization coupled with time-of-flight analyzers ("MALDI-TOF") permit the analysis of analytes at femtomole levels in very short ion pulses. Mass spectrometers that combine time-of-flight analyzers with tandem MS are also well known to the artisan. Additionally, multiple mass spectrometry steps may be combined in methods known as "MS/MS". Various other combinations may be employed, such as MS/MS/TOF, MALDI/MS/MS/TOF, or SELDI/MS/MS/TOF mass spectrometry.

In particular, since most of the ligands are metals, ICP-MS may be preferred. Inductively coupled plasma mass spectrometry (ICP-MS) is a type of mass spectrometry which is capable of detecting metals and several non-metals at concentrations as low as one part in 10¹⁵ (part per quadrillion, ppq) on non-interfered low-background isotopes. This is achieved by ionizing the sample with inductively coupled plasma and then using a mass spectrometer to separate and quantify those ions. Inductively coupled plasma optical emission spectrometry (ICP-OES), is an analytical technique used for the detection of chemical elements. It is a type of emission spectroscopy that uses the inductively coupled plasma to produce excited atoms and ions that emit electromagnetic radiation at wavelengths characteristic of a particular element. It is a flame technique with a flame temperature in a range from 6000 to 10000 K. The intensity of this emission is indicative of the concentration of the element within the sample.

One or more steps of the methods may be performed using automated machines. In particular, one or more purification steps are performed on-line, and more preferably all of the LC purification and mass spectrometry steps may be performed in an on-line fashion.

Typically, the predetermined reference value is a threshold value or a cut-off value, which can be determined experimentally, empirically, or theoretically. A threshold value can also be arbitrarily selected based upon the existing experimental and/or clinical conditions, as would be recognized by a person of ordinary skilled in the art. For example, retrospective measurement of the binding capacity in properly banked historical samples may be used in establishing the predetermined reference value. The threshold value has to be determined in order to obtain the optimal sensitivity and specificity according to the function of the test and the benefit/risk balance (clinical consequences of false positive and false negative). Typically, the optimal sensitivity and specificity (and so the threshold value) can be determined using a Receiver Operating Characteristic (ROC) curve based on experimental data. For example, after determining the binding capacity in a group of reference, one can use algorithmic analysis for the statistic treatment of the expression levels determined in samples to be tested, and thus obtain a classification standard having significance for sample classification.

The full name of ROC curve is receiver operator characteristic curve, which is also known as receiver operation characteristic curve. It is mainly used for clinical biochemical diagnostic tests. ROC curve is a comprehensive indicator that reflects the continuous variables of true positive rate (sensitivity) and false positive rate (1-specificity). It reveals the relationship between sensitivity and specificity with the image composition method. A series of different cut-off values (thresholds or critical values, boundary values between normal and abnormal results of diagnostic test) are set as continuous variables to calculate a series of sensitivity and specificity values. Then sensitivity is used as the vertical coordinate and specificity is used as the horizontal coordinate to draw a curve. The higher the area under the curve (AUC), the higher the accuracy of diagnosis. On the ROC curve, the point closest to the far upper left of the coordinate diagram is a critical point having both high sensitivity and high specificity values. The AUC value of the ROC curve is between 1.0 and 0.5. When AUC>0.5, the diagnostic result gets better and better as AUC approaches 1. When AUC is between 0.5 and 0.7, the accuracy is low. When AUC is between 0.7 and 0.9, the accuracy is moderate. When AUC is higher than 0.9, the accuracy is high. This algorithmic method is preferably done with a computer. Existing software or systems in the art may be used for the drawing of the ROC curve, such as: MedCalc 9.2.0.1 medical statistical software, SPSS 9.0, ROCPOWER.SAS, DESIGNROC.FOR, MULTIREADER POWER.SAS, CREATE-ROC.SAS, GB STAT VI0.0 (Dynamic Microsystems, Inc. Silver Spring, Md., USA), etc.

In particular, a score which is a composite of the measured binding capacities is determined and compared to a reference value wherein a difference between said score and said reference value indicates whether the subject suffers from a liver dysfunction. As used herein, the term "score" refers to a piece of information, usually a number that conveys the result of the subject on a test. A risk scoring system separates a patient population into different risk groups; herein the process of risk stratification classifies the patients into very high-risk, high-risk, intermediate-risk and low-risk groups.

In particular, the method of the disclosure comprises the use of a classification algorithm. As used herein, the term "algorithm" is any mathematical equation, algorithmic, analytical or programmed process, or statistical technique that takes one or more continuous parameters and calculates an output value, sometimes referred to as an "index" or "index value." Non-limiting examples of algorithms include sums, ratios, and regression operators, such as coefficients or exponents, biomarker value transformations and normalizations (including, without limitation, those normalization schemes based on clinical parameters, such as gender, age, or ethnicity), rules and guidelines, statistical classification models, and neural networks trained on historical populations. Of particular use in combining parameters are linear and non-linear equations and statistical classification analyses to determine the relationship between levels of said parameters and the risk of allograft loss. Of particular interest are structural and syntactic statistical classification algorithms, and methods of risk index construction, utilizing pattern recognition features, including established techniques such as cross-correlation, Principal Components Analysis (PCA), factor rotation, Logistic Regression (LogReg),Linear Discriminant Analysis (LDA), Eigengene Linear Discriminant Analysis (ELDA), Topological Data Analysis (TDA), Neural Networks, Support Vector Machine (SVM) algorithm and Random Forests algorithm (RF), Recursive Partitioning Tree (RPART), as well as other related decision tree classification techniques, Shrunken Centroids (SC), StepAIC, Kth-Nearest Neighbor, Boosting, Decision Trees, Neural Networks, Bayesian Networks, Support Vector Machines, Recommender System Algorithm and Hidden Markov Models, among others. Other techniques may be used in survival and time to event hazard analysis, including Cox, Weibull, Kaplan-Meier and Greenwood models well known to those of skill in the art.

As used herein, the term "classification algorithm" has its general meaning in the art and refers to classification and regression tree methods and multivariate classification well known in the art such as described in US 8,126,690; WO2008/156617.

In particular, the method of the present disclosure comprises the use of a machine learning algorithm. The machine learning algorithm may comprise a supervised learning algorithm. Examples of supervised learning algorithms may include Average One-Dependence Estimators (AODE), Artificial neural network (e.g., Backpropagation), Bayesian statistics (e.g., Naive Bayes classifier, Bayesian network, Bayesian knowledge base), Case-based reasoning, Decision trees, Inductive logic programming, Gaussian process regression, Group method of data handling (GMDH), Learning Automata, Learning Vector Quantization, Minimum message length (decision trees, decision graphs, etc.), Lazy learning, Instance-based learning Nearest Neighbor Algorithm, Analogical modeling, Probably approximately correct learning (PAC) learning, Ripple down rules, a knowledge acquisition methodology, Symbolic machine learning algorithms, Subsymbolic machine learning algorithms, Support vector machines, Random Forests, Ensembles of classifiers, Bootstrap aggregating (bagging), and Boosting (e.g. XGBoost). Supervised learning may comprise ordinal classification such as regression analysis and Information fuzzy networks (IFN). Alternatively, supervised learning methods may comprise statistical classification, such as AODE, Linear classifiers (e.g., Fisher's linear discriminant, Logistic regression, Naive Bayes classifier, Perceptron, and Support vector machine), quadratic classifiers, k-nearest neighbor, Boosting, Decision trees (e.g., C4.5, Random forests), Bayesian networks, and Hidden Markov models. The machine learning algorithms may also comprise an unsupervised learning algorithm. Examples of unsupervised learning algorithms may include artificial neural network, Data clustering, Expectation-maximization algorithm, Self-organizing map, Radial basis function network, Vector Quantization, Generative topographic map, Information bottleneck method, and IBSEAD. Unsupervised learning may also comprise association rule learning algorithms such as Apriori algorithm, Eclat algorithm and FP-growth algorithm. Hierarchical clustering, such as Single-linkage clustering and Conceptual clustering, may also be used. Alternatively, unsupervised learning may comprise partitional clustering such as K-means algorithm and Fuzzy clustering. In some instances, the machine learning algorithms comprise a reinforcement learning algorithm Examples of reinforcement learning algorithms include, but are not limited to, temporal difference learning, Q-learning and Learning Automata. Alternatively, the machine learning algorithm may comprise Data Pre-processing. In particular, the boosting model includes the XGBoost algorithm.

Thus, in particular, the method of the present disclosure comprises a) measuring the binding capacities of gold (Au), copper (Cu), cadmium (cd), L-thyroxine and dansylsarcosine; b) implementing a classification algorithm on data comprising the measured binding capacities so as to obtain an algorithm output; c) determining the probability that the subject suffers from a liver dysfunction.

In particular, the algorithm is implemented on a computer using well-known computer processors, memory units, storage devices, computer software, and other components. Typically, the computer contains a processor, which controls the overall operation of the computer by executing computer program instructions which define such operation. The computer program instructions may be stored in a storage device (e.g., magnetic disk) and loaded into memory when execution of the computer program instructions is desired. The computer also includes other input/output devices that enable user interaction with the computer (e.g., display, keyboard, mouse, speakers, buttons, etc.). One skilled in the art will recognize that an implementation of an actual computer could contain other components as well.

In particular, the algorithm is implemented using computers operating in a client-server relationship. Typically, in such a system, the client computers are located remotely from the server computer and interact via a network. The client-server relationship may be defined and controlled by computer programs running on the respective client and server computers. In particular, the results may be displayed on the system for display, such as with LEDs or an LCD. Accordingly, in particular, the algorithm can be implemented in a computing system that includes a back-end component, e.g., as a data server, or that includes a middleware component, e.g., an application server, or that includes a front-end component, e.g., a client computer having a graphical user interface or a Web browser through which a user can interact with an implementation, or any combination of one or more such back-end, middleware, or front-end components. The components of the system can be interconnected by any form or medium of digital data communication, e.g., a communication network. Examples of communication networks include a local area network ("LAN") and a wide area network ("WAN"), e.g., the Internet. The computing system can include clients and servers. A client and server are generally remote from each other and typically interact through a communication network. The relationship of client and server arises by virtue of computer programs running on the respective computers and having a client-server relationship to each other.

In particular, the algorithm is implemented within a network-based cloud computing system. In such a network-based cloud computing system, a server or another processor that is connected to a network communicates with one or more client computers via a network. A client computer (e.g. a mobile device, such as a phone, tablet, or laptop computer) may communicate with the server via a network browser application residing and operating on the client computer, for example. A client computer may store data on the server and access the data via the network. A client computer may transmit requests for data, or requests for online services, to the server via the network. The server may perform requested services and provide data to the client computer(s). The server may also transmit data adapted to cause a client computer to perform a specified function, e.g., to perform a calculation, to display specified data on a screen, etc. For instance, the physician may register the parameters (i.e. input data) on, which then transmits the data over a long-range communications link, such as a wide area network (WAN) through the Internet to a server with a data analysis module that will implement the algorithm and finally return the output (e.g. score) to the mobile device.

In particular, the output results can be incorporated in a Clinical Decision Support (CDS) system. These output results can be integrated into an Electronic Medical Record (EMR) system.

A further object of the present disclosure relates to a kit or device for performing the method of the present disclosure, comprising means for determining the binding capacities as described above. In particular, the kits or devices of the present disclosure comprise at least one sample collection container for sample collection. Collection devices and container include but are not limited to syringes, lancets, BD VACUTAINER^{®} blood collection tubes. In particular, the container contains a predetermined amount of the ligand. In particular, the kits or devices described herein further comprise instructions for using the kit or device and interpretation of results. In particular, the kit or device of the present disclosure further comprises a microprocessor to implement an algorithm so as to determine the probability that the patient suffers from a liver dysfunction. In particular, the kit or device of the present disclosure further comprises a visual display and/or audible signal that indicates the probability determined by the microprocessor. In particular, the kit or device of the present disclosure comprises: i) a mass spectrometer; ii) a receptacle into which the serum sample is placed, and which is connectable to the mass spectrometer so that the mass spectrometer can quantify the amount of the free ligand; iii) optionally a microprocessor to implement an algorithm on data so as to determine the probability that the subject suffers from a liver dysfunction and iv) a visual display and/or audible signal that indicates the probability determined by the microprocessor.

The disclosure will be further illustrated by the following figures and examples.

### FIGURES:

**Figure 1****. (A-D) Serum enhanced binding of Cu, Cd, Au and dansylsarcosine.**
**Figure 2****. (A-D) Discrimination between control patients with no liver disease and cirrhotic patients by the SEB test.** Ligands were spiked to serums using solutions at the following concentrations, expressed as HSA/ligand ratios (namely 1 molecule of HSA for X atoms or molecules of ligand): Cu 1/10, L-thyroxine 1/10, Au 1/100, dansylsarcosine 1/5. Cu was tested in 18 patients, Au in 16 patients, L-thyroxine in 16 patients and dansylsarcosine in 6 patients only. The ordinates represent the (µM of free ligand)/(µM of HSA) ratio.
**Figure 3****. Determination of HSA isoforms in 18 cirrhotic and 18 control patients.** Observation of high abundances of HSA isoforms in all cirrhotic patients.
**Figure 4****. (A-D) Discrimination between non-cirrhotic and cirrhotic patients by the SEB test with lower concentrations for Au, Cu and L-thyroxine.** Cd was also tested in this group at a ratio of 1/5 (HAS/Cd). ** means p< 0.001. n= 12 for all ligands in both groups except L-thyroxine where n=6.
**Figure 5****. (A-D) Time evolution of ligand binding in rats after daily administration for 1, 3, 7, 10 and 14 days of high doses of ethanol.** The groups of rats (n=9 each) D1, D3, D7, D10 and D14 received 0.4 g of ethanol for 1, 3, 7, 10 and 14 days respectively. * = p < 0.05 ; ** p < 0.01.
**Figure 6****. (A-D) Relative abundances of albumin isoforms in all the groups of rats.** Alb-Acet, Alb-Cys, Alb-Gly, and Alb-Glut stands for acetylated albumin, cysteinylated albumin, glycosylated albumin and glutathione-conjugated albumin respectively. * = p < 0.05; ** p < 0.01.
**Figure 7****. (A-E) Discrimination between control patients with no liver disease and cirrhotic patients by SEB test in the development cohort.**
**Figure 8****. Determination of the 3 most abundant HSA isoforms in 45 cirrhotic and 45 control patients.**

### EXAMPLE: The "Serum Enhanced Binding" test as a biomarker of liver dysfunction

### Methods

### Chemicals:

The following reagents were purchased form Sigma-Aldrich and used to prepare the ligands solutions: cobalt(II) chloride (CAS : 7646-79-9), gold(III) chloride trihydrate (CAS : 16961-25-4), copper(II) chloride (CAS : 7447-39-4), silver acetate (CAS : 563-63-3), L-thyroxine sodium salt pentahydrate (CAS : 6106-07-6). Dansylsarcosine Piperidinium Salt >95%, was purchased from RareChemicals GmbH. All ligands solutions were prepared in MiliQ purified water. Albumin Vialebex^{®}, 200mg/mL was used to test HSA binding capacity.

### Patients and samples

Patient samples were all from blood leftovers of biochemistry laboratory tests prescribed according to the standard of care. In accordance with French regulations and Good Clinical Practice for biomedical studies, patients were informed of, and were able to oppose to, the use of the leftovers of their blood samples at any time (CSP article L1211-2).

The cohort was composed of cirrhotic patients and of patients with no liver dysfunction as controls. Patients were considered as free from liver dysfunction on the basis of their clinical diagnosis and their liver function biochemical tests, namely, aspartate transaminase, alanine transaminase, alkaline phosphatase, 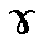 -glutamyltransferase, free and total bilirubin and albumin.

Cirrhotic patients were included based on the gastroenterologists' diagnosis, their liver function biochemical tests and their Child-Pugh scores.

Plasmas or serums were obtained by centrifugation of blood at 3000 rpm for 10 min at 4°C. For the cohort, the SEB test was performed within 24h of the biochemical tests. When volume permitted, plasma or serum samples were then stored at -20°C for stability tests.

HSA isoforms were determined for all patients, as described below.

### Study of the binding capacities of HAS in patients with no hepatic dysfunction:

In a first step, we have evaluated separately the global capacity of serum to bind Cu, Au, L-thyroxine, Cd and dansylsarcosine in patients with no liver dysfunctions. Increasing concentrations of each ligand were added to patient serum samples in order to obtain HSA/ligand theoretical ratios (mol/mol) of 1/1, 1/5, 1/10, 1/20, 1/50, 1/100, 1/500 and 1/1000 when possible. These theoretical ratios were calculated on the basis of HSA blood concentration of 0.6 mM, which is an average concentration in healthy subjects.

Six different serums (from six different patients) per ratio and per ligand were used for this evaluation. After incubation for 30 min of the serum samples spiked with a ligand, they were ultracentrifugated to measure the unbound ligand in the ultrafiltrates. In details,
Serum (200 µL) was incubated for 30 min at 4°C with the abovementioned ligands with different solutions concentrations (500 µL of solutions at increasing concentrations of ligands),
The incubated serum was ultrafiltrated on Amicon^{®} filters with a 30 kDa cut-off,
The ultrafiltrate (10 µL) was then diluted in HNO₃ 0.1 M before analysis using a multi-element ICP-MS method for the determination of free (unbound) ligand concentrations. The ICP-MS method measured Cu, Au, Cd, iodine (for L-Thyroxine) and sulfur (for dansylsarcosine) separately or simultaneously, depending on the sample content.

Percentages of retained ligands quantity as well as the real ratios of HSA/bound ligands (mol/mol) were then calculated, since the actual HSA concentration in each serum was known.

This allowed us to determine the maximum capacity of the serum to bind each ligand. These ceilings are at the basis of the SEB test to discriminate serum with modified HSA forms from serum with mostly native HSA.

### Comparison of binding capacities of HSA in cirrhotic patients and controls (patients without hepatic dysfunction)

### Discovery cohort

After setting thresholds corresponding to the retention of more than 90% of each ligand, we performed the SEB test on the serum of patients with diagnosed cirrhosis (n=18) as compared to patients with no liver dysfunctions (n=18). The SEB test was then performed as described above but with the different solutions containing ligands, at specific concentrations proportional to the ligands' binding threshold. Briefly, solutions of Cu, Au, dansylsarcosine and L-thyroxine were prepared at 5950 µM, 23800 µM, 11900 µM and 150 µM, respectively. The solutions were incubated separately with 200 µL of serum for Cu, Au, and dansylsarcosine and with 50 µL of serum for L-thyroxine.

Albumin isoforms were determined in all serum samples of these two groups as described below.

In another experiment, we analyzed serum samples from 12 cirrhotic patients and 12 controls in order to study the discrimination power of the test when using solutions of ligands at lower concentrations. For this, Cu, Cd and Au solutions concentrations were set at 1190 µM for Cu, 1190 µM for Cd, 11900 µM for Au and 75 for L-thyroxine 75 µM.

### Development cohorts

The SEB test and the identification of the most important isoforms of HSA, namely, HSA-Cys, HAS-Gly and HAS-Cys-Gly, were performed as described previously in a development cohort including 45 cirrhotic patients and 45 patients with no liver impairments. The statistical analysis were performed to discriminate patients in a first step then to discriminate patients upon their Child-Pugh scores (Class A (CA): "least severe liver disease", Class B (CB): "moderately severe liver disease", Class C (CC): "most severe liver disease"). The dataset was split up in a training (75%) and a testing (25%) dataset using random table. Prediction models were developed using extreme gradient boosting (package R xgboost v0.90.0.2) including 2 repetitions of 3 groups cross validations for isoform alone or for ligands of SEB test alone. Global discrimination capacity of disease vs non disease and discrimination for each Child-Pugh stage of cirrhosis were tested. The performances were evaluated in the testing data and the confusion matrix was drawn as well as global accuracy and its 95%CI.

### Animal model:

We also set up an animal experiment to investigate the time and severity of liver dysfunction at which the test turns positive. In this experimentation, high doses of ethanol were used to induce liver injuries in six groups of male Wistar rats (Janvier Labs, France). Each group contained 6 to 9 rats. Two ml of a solution of 50% of ethanol (0.4 g of ethanol) was administrated orally for 1 day in the 1^{st} group, for 3 days in the 2^{nd} group, for 7 days in the 3^{rd}, for 10 days in the 4^{th} group and for 14 days in the 5^{th} group. Blood and liver were collected from the sacrificed rats 24h after the last ethanol administration. A control group (n=9) received oral administration or a saline solution for 14 days and rats were sacrificed and sampled at day 15. The SEB test was applied to the rats of all these groups. Albumin isoforms, as described below, were also determined for all the groups.

### ICP-MS analysis

Calibration curves were built with 6 calibrants for each element. Concentrations ranged between 10 and 100 µg/L for Cu, Cd, Au and sulfur and between 1 and 20 µg/L for L-thyroxine.

L-cystein was used for the calibration of sulfur and L-thyroxine for the calibration of iodine. Cu was measured Cu at *m*/*z* 65, Cd at *m*/*z* 112, Au at *m*/*z* 197, iodine at *m*/*z* 127 and sulfur at *m*/*z* 48 as described in EL BALKHI et al. 2010 [9]. To be able to measure sulfur (³²S), interfered by ³²O₂, we introduced oxygen as a reactant gas in the reaction cell of the instrument to generate ⁴⁸SO. For this, the kinetic energy discrimination (KED) mode was used with oxygen flow rate at 0.3 ml/min. This was applied for all element measurements and for all calibration points, controls and ultrafiltrates. The ultrafiltrates were diluted with HNO₃ 0.1 M when necessary.

### HSA isoforms determinations:

To study the albumin modifications in all samples, analysis was carried out using micro-liquid chromatography coupled to high resolution Q-TOF mass spectrometry (TripleTOF^{®} 5600+, Sciex). Plasma or serum samples from all studied groups were diluted with ultrapure water to 1:1,000 (v:v) and 5 µL of the diluted serum were injected. A C4 Chrom XP (100X0,3 mm; 3 µm) Eksigent column was used for the chromatographic separation of albumin isoforms, together with a mobile phase solvent A (0.1% formic acid in ultrapure water) and solvent B (0.1% formic acid in acetonitrile). The analysis was performed in gradient mode, programmed as follows: 0-1 min, 20% B; 1-5 min, 20% to 50% B; 5-6 min, 50% to 95% B; 6-8 min, 95% B; 8-8.5 min; 95% to 20% B; 8.5-13 min, equilibration with 20% B. The run lasted 13 min and the total flow rate was kept constant at 5 µL/min.

All MS parameters were controlled by Analyst^{®} TF 1.7 (Sciex). m/z ratios were first scanned from m/z 400 and 1250 using the TOF MS scan mode with an accumulation time of 2 s. The albumin spectra obtained were then deconvoluted within the mass range from 66,000 Da to 67,000 Da with PeakView 2.1 software (Sciex). From the intensity of the peak, the relative abundance of albumin isoforms was calculated relative to the intensity of native albumin.

The same method of isoform determination was applied to rat serum obtained from the animal experiment.

### Results

### Enhanced binding capacity of serum/HSA

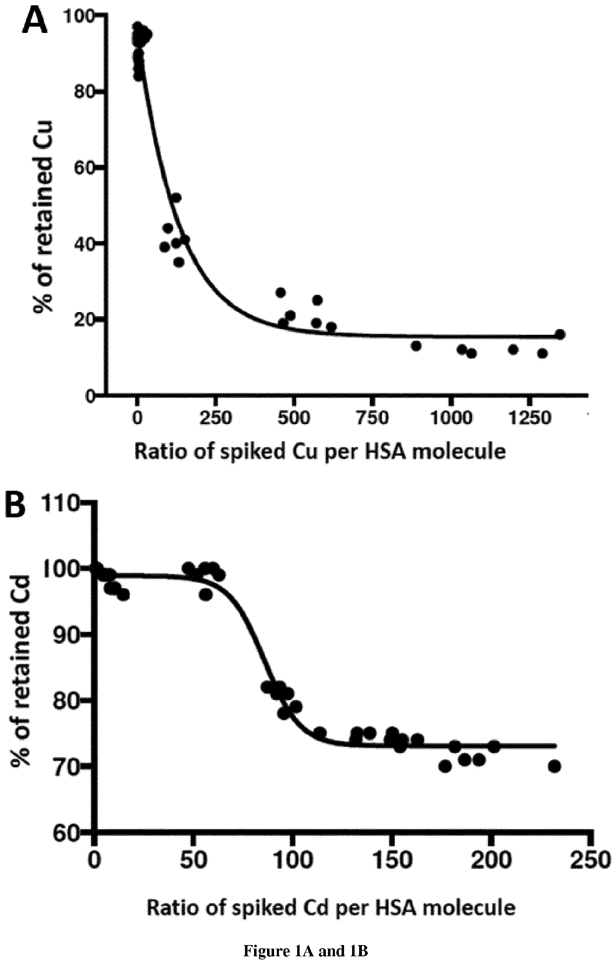

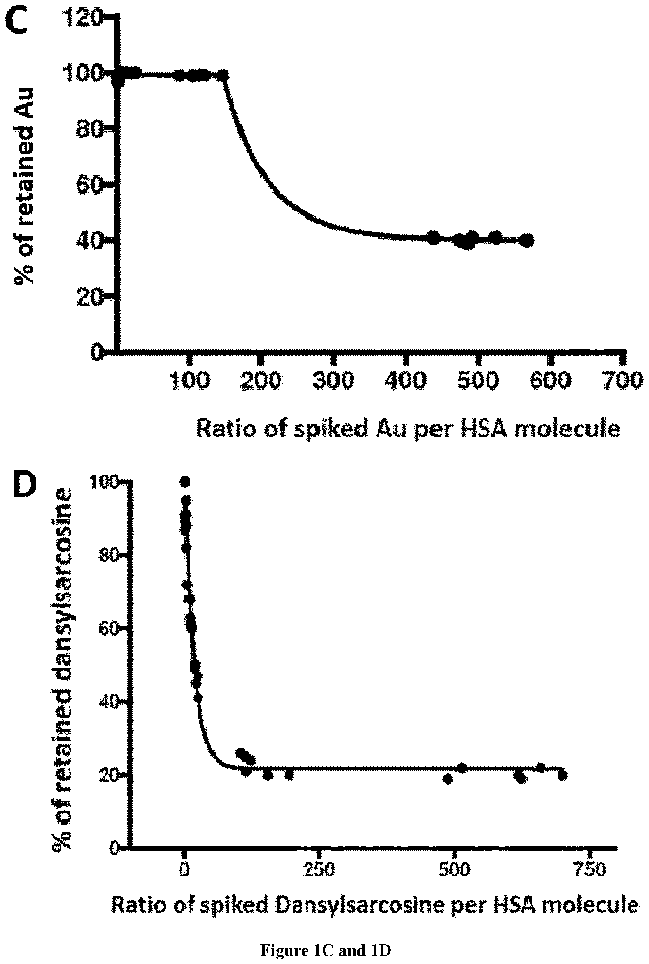

By adding increasing concentrations of Cu to serum, we observed that up to 12 Cu atoms per albumin molecule were retained on the ultracentrifugation filter with an average retention of 95%. This percentage dropped to 40% or less when more Cu was added **(****Figure 1A to 1D****).** Serum samples were able to bind with 100% retention up to 150 atoms of Au, 50 atoms of Cd and 2.5 molecules of dansylsarcosine per molecule of albumin. Serum samples were able to bind at least 10 molecules of L-thyroxine with 100% retention, but L-thyroxine could not be tested above the 1/10 ratio (HSA/L-thyroxine) because of dissolution problems. In order to confirm that the binding is only due to HAS and that there is no unspecific binding to other serum proteins, we performed the same tests with the Vialebex^{®} commercial albumin solution at 200mg/mL (supplemental data). The binding capacities of commercial solution of pure HSA were equivalent or higher than those of patient serum: for instance, the Cu/HSA retention ratio was 40 and the Au/HSA 150.

Based on these results we set thresholds best able to discriminate native HSA from modified HSA. Solutions of Au, Cu, dansylsarcosine and L-thyroxine were then prepared to obtain theoretical ratios of 1/100, 1/10, 1/5, and 1/10, respectively. The solutions were then incubated with serums samples from cirrhotic and control patients, as described above.

### Comparison of serum enhanced binding capacities in cirrhotic and patients with no liver dysfunction

### Discovery cohort

Among the 18 cirrhotic patients, cirrhosis was due to alcohol alone in 8 patients, to alcohol and VHC in 1 case, to a metabolic syndrome (NASH) in 5 cases, alcohol and NASH in 3 cases, alcohol, NASH and viral infection in 1 case. Albumin concentrations ranged between 18.2 and 34 g/L. Child-Pugh scores for all patients are shown in **Table 1.**

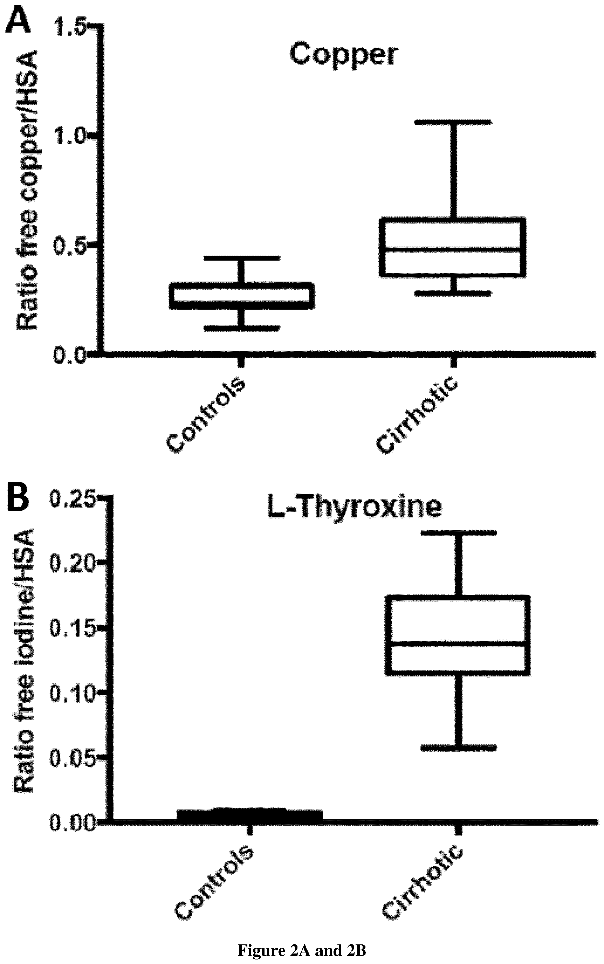

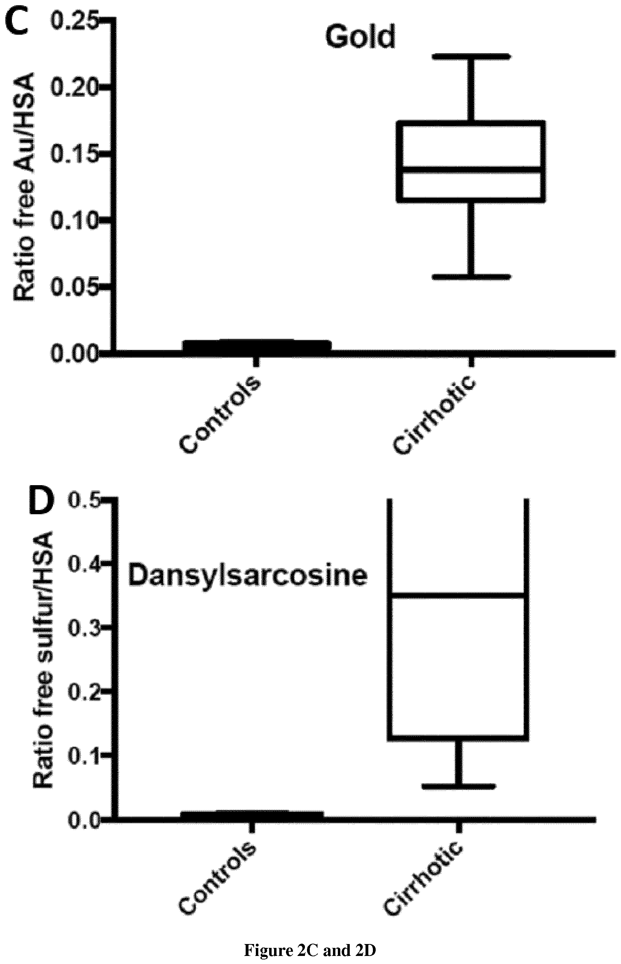

Au, dansylcarcosine, and L-thyroxine were able to discriminate with 100% specificity and sensitivity cirrhotic patients from control patients, as shown in **Figure 2A to 2D**. Cu was able to discriminate cirrhotic patients with 72 % specificity but with 100% sensitivity.

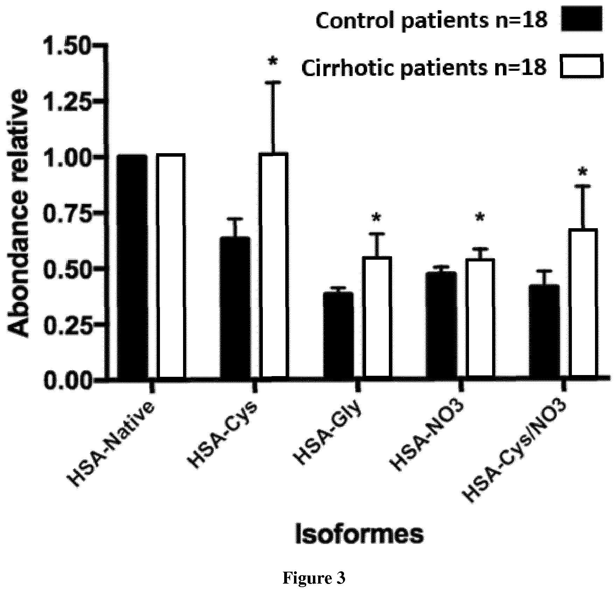

All the 18 cirrhotic patients and the 18 control patients were analyzed to determine the abundance of HSA isoforms in their serum. We observed high abundances of HSA isoforms in all cirrhotic patients with the presence of significantly increased cysteinylated HSA (HSA-Cys), Glycated HSA (HSA-Gly), nitrosylated HSA (HSA-NO3) and cysteinylated and nitrosylated HSA (HSA-Cys/NO3), as shown in **Figure 3****.**

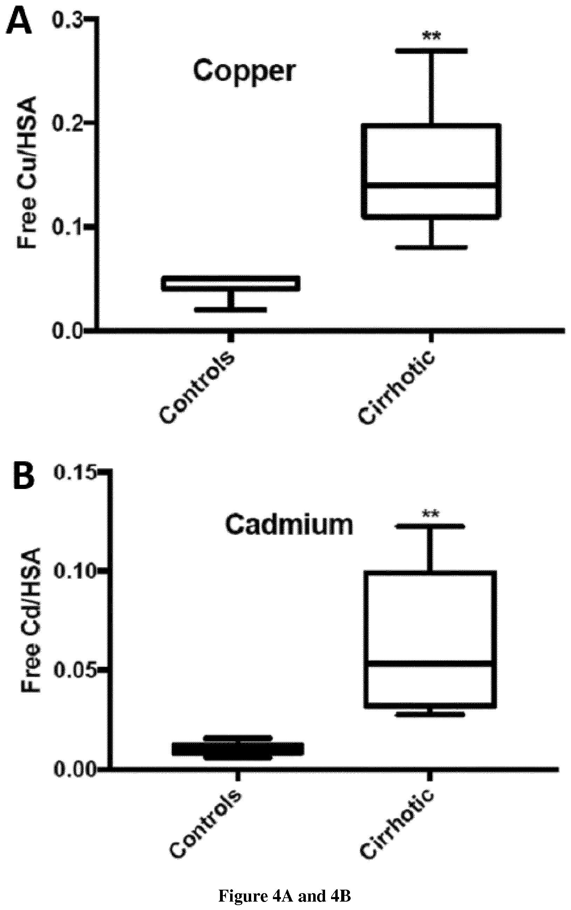

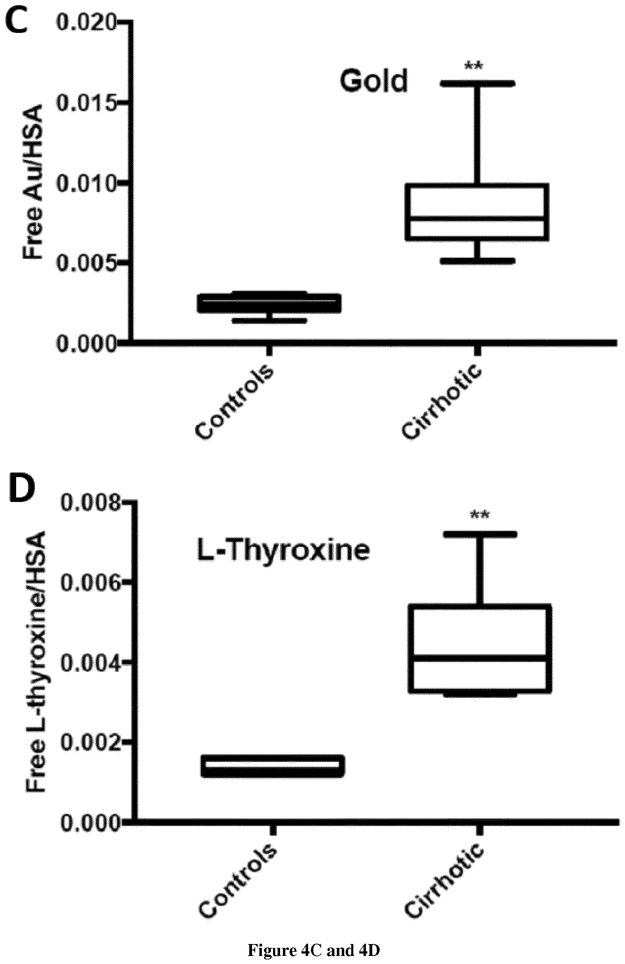

In a second step, 12 cirrhotic patients and 12 control patients were then included to test lower concentrations for Cu, Au and L-thyroxine (1/5, 1/50 and 1/5, respectively). Additionally, Cd was tested in this group at a ratio of HSA/Cd of 1/5. All the ligands were able to discriminate cirrhotic patients with 100% sensitivity and specificity as shown in **Figure 4A to 4D****.**

### Development cohort

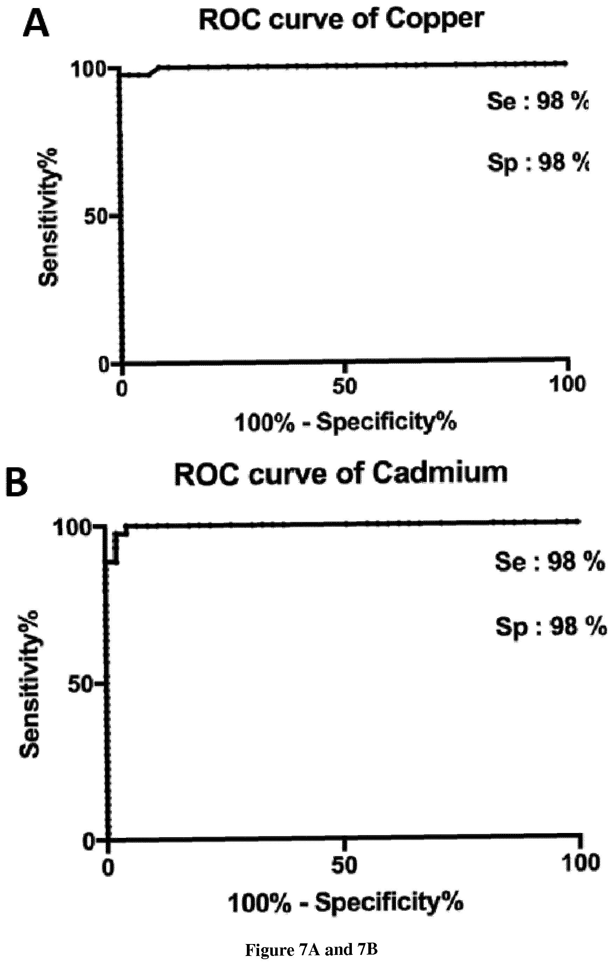

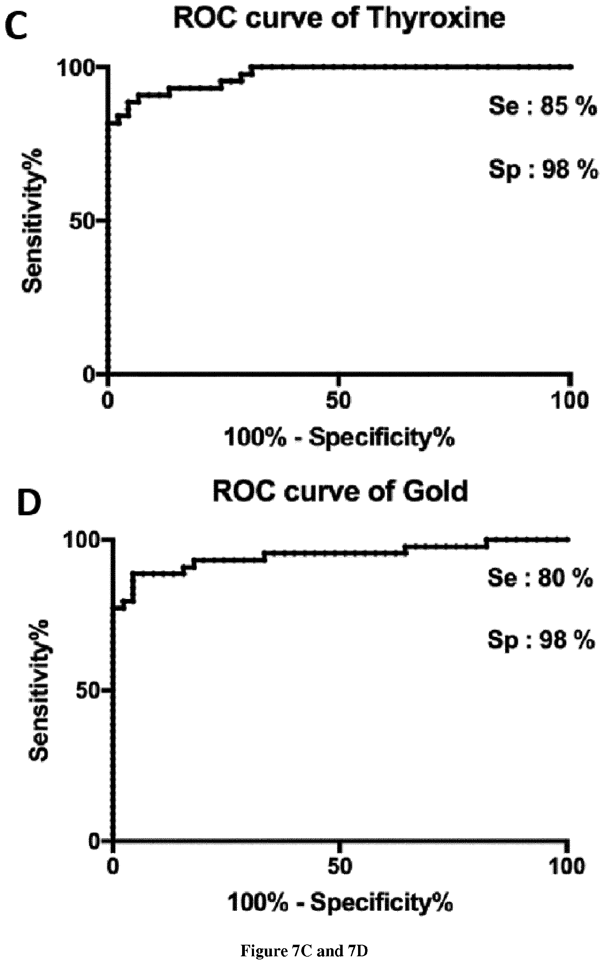

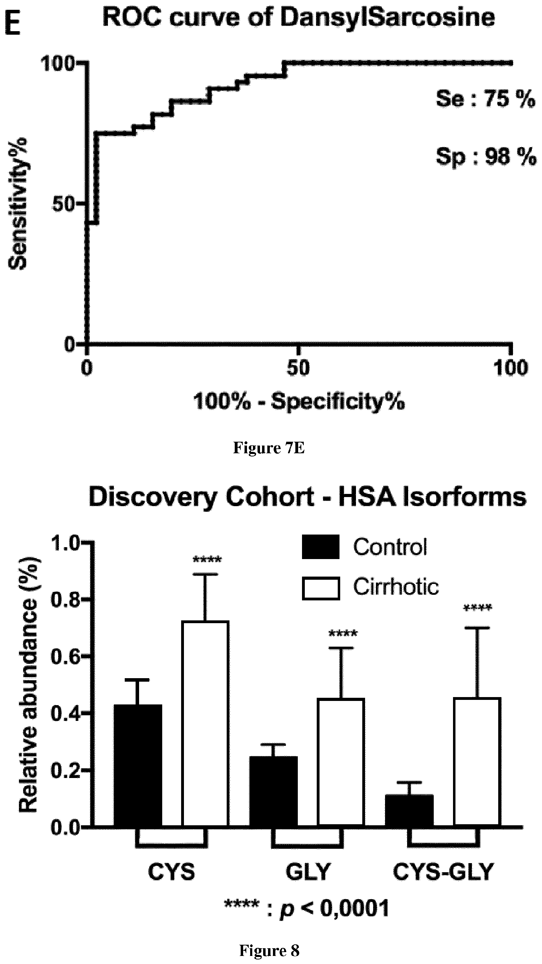

As shown in **Figure 7A to 7E**, the performances of the SEB test ligands were consistent with the previous results: the specificity and sensitivity of Cu and Cd were 98%; L-thyroxine offered Se 85% and Sp 98%; Au Se and Sp were 80 and 98%. However, dansylsarcosine Se and Sp were only of 75% and 98%, respectively. When all the ligands were taken together in a principal component analysis, the group of cirrhotic patients was visibly well separated from control patients (data not shown). The 3 principal HSA isoforms were significantly higher in cirrhotic patients in comparison with control patients as shown in **Figure 8****.**

The cohort included 45 cirrhotic patients and 45 patients with no liver dysfunctions. Among the 45 cirrhotic patients, 6 were removed due to absence of formal classification of the disease but these patients were used to be predicted by the final models. So the training set included 65 patients (12CA, 11CB, 6CC and 36 N) and the testing 19 patients (3 CA, 3 CB, 1 CC and 12 N).

### Uniclass model for SEB test ligands: cirrhosis vs no cirrhosis

The parameters of the best model for "ligands uniclass" were nrounds=5 (number of passes on the data), max_depth=2 (Maximum depth of a tree), eta=0.3 (learning rate), colsample_bytree=0.25 (is the subsample ratio of columns when constructing each tree), subsample=0.5 (Subsample ratio of the training instances).

Performance in the testing dataset were excellent with an accuracy (CI95%)=1 (0.82,1). Seven cirrhotic patients and 12 patients with no liver dysfunctions were well predicted with no false positive or false negative.

### Uniclass model for HSA isoform: cirrhosis vs no cirrhosis

The parameters of the best model for "metals uniclass" were nrounds=5 (number of passes on the data), max_depth=2 (Maximum depth of a tree), eta=0.3 (learning rate), colsample_bytree=0.25 (is the subsample ratio of columns when constructing each tree), subsample=0.5 (Subsample ratio of the training instances).

Performance in the testing dataset were excellent with an accuracy (CI95%)=0.95 (0.74, 0.999). Among the 12 patients with no liver impairments, 12 were well predicted and only one cirrhotic patient was not well predicted.

### Multiclass model for SEB test ligands

The parameters of the best model for "metals multiclass" were nrounds=5 (number of passes on the data), max_depth=2 (Maximum depth of a tree), eta=0.2 (learning rate), colsample_bytree=0.5 (is the subsample ratio of columns when constructing each tree), subsample=1 (Subsample ratio of the training instances).

Performance in the testing dataset offered an accuracy (CI95%)=0.79 (0.54, 0.94). The confusion matrix was as follows:

### Reference

| | | | Reference | | |
|---|---|---|---|---|---|
| Prediction | | CA | CB | CC | N |
| | CA | 1 | 1 | 1 | 0 |
| | CB | 2 | 2 | 0 | 0 |
| | CC | 0 | 0 | 0 | 0 |
| | N | 0 | 0 | 0 | 12 |

### Model for isoform multiclass: prediction of Child-Push scores

The parameters of the best model for "isoforms multiclass" were nrounds=5 (number of passes on the data), max_depth=2 (Maximum depth of a tree), eta=0.1 (learning rate), colsample_bytree=1 (is the subsample ratio of columns when constructing each tree), subsample=1 (Subsample ratio of the training instances).

Performance of HSA isoforms in the testing dataset showed an accuracy (CI95%)=0.84 (0.60,0.97). Three cirrhotic patients out of 7 were not well predicted.

### Animal experiment

After daily administration of 0.4 g of ethanol (1.6 g ethanol/kg of body weight) to the different groups of rats, we observed a significant increase of AST in the groups receiving ethanol for more than 7 days. After 10 days of ethanol administrations ALT was significantly higher than in the control group. Alkaline phosphatase (ALP), free and total bilirubin were unchanged in comparison to controls **(Table 2).** Histological tests on the liver of rats of group D14 showed a very slight fibrosis (data not shown). No liver tissue damages were visible in the other groups.

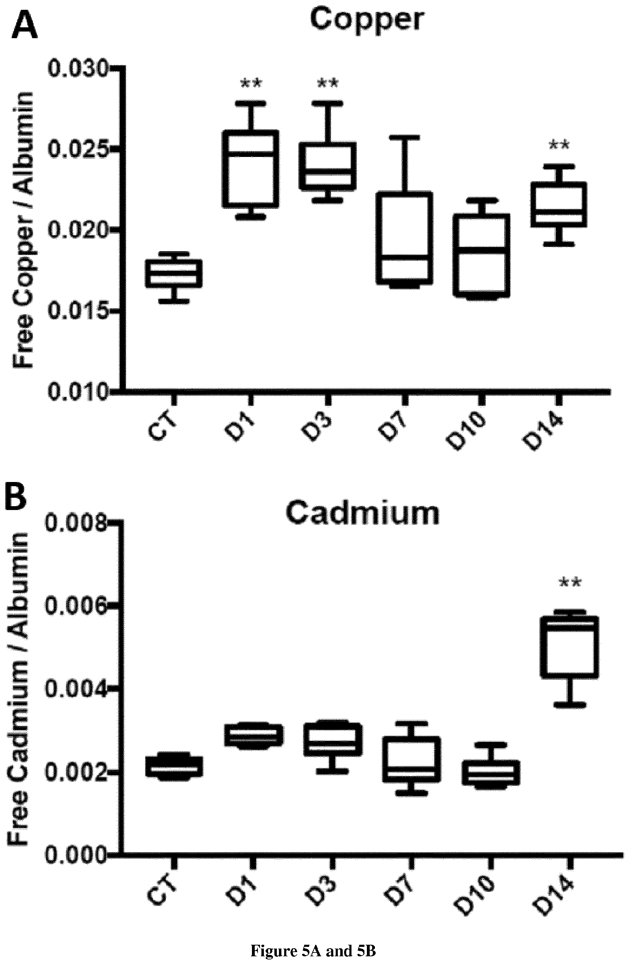

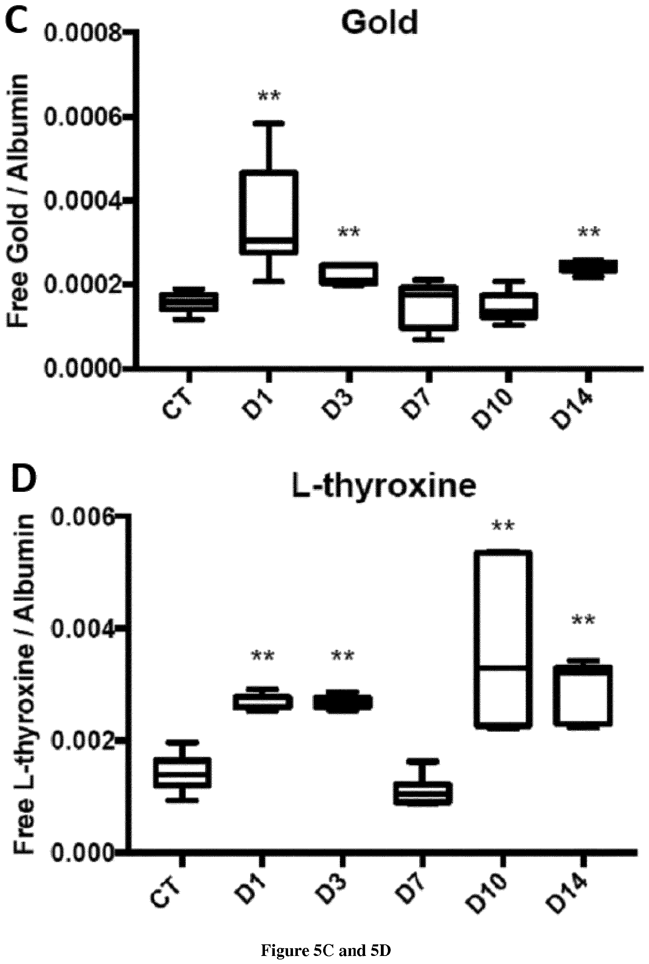

The SEB test was performed in the serum of all groups of rats using Cu, Cd, L-thyroxine at thresholds 1/5 and Au at a threshold 1/50, as described above. As shown in **Figure 5A to 5D****,** all rats in the group D14 were positive for all tested ligands. Rat serum had decreasing binding capacities for Au after the first day of administration but this capacity was restored in the group D7. The same profile was observed for the biding capacity of Cu and L-thyroxine. However, the binding capacity of Cd was only decreased in the group D14.

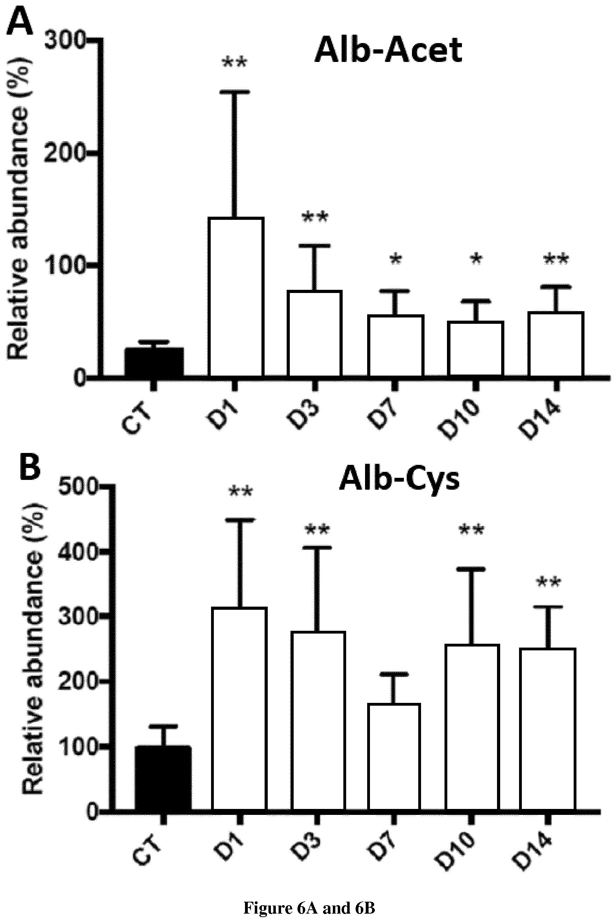

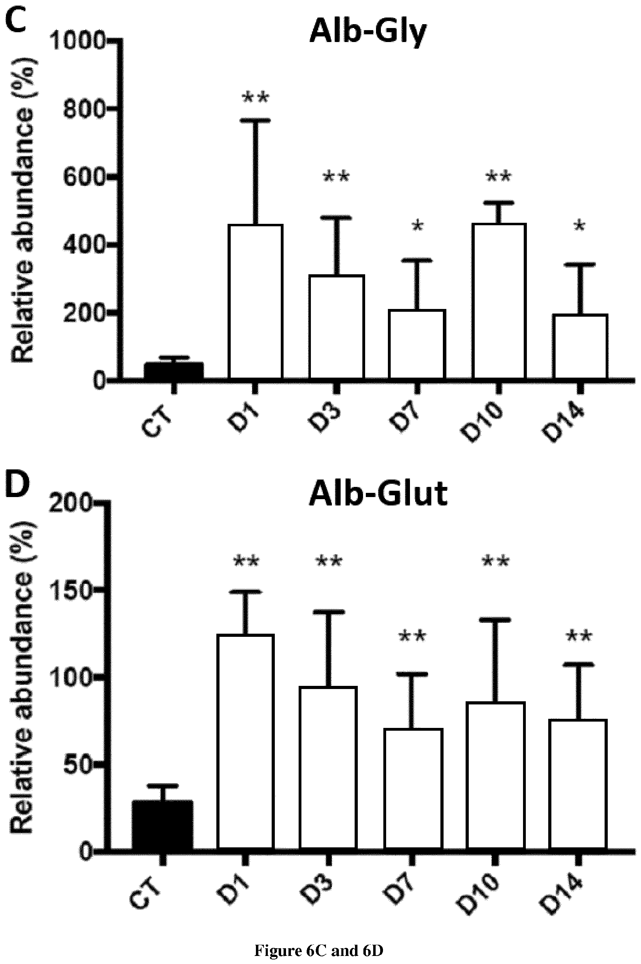

Micro-LC- HRMS showed significant increases of all the identified albumin isoforms in these groups of rats. As depicted in **Figure 6A to 6D****,** acetylated albumin (Alb-Acet), glycosylated albumin (Alb-Gly), and gluthation-conjugated albumin increased very rapidly showing significant differences between groups. The cysteinylated albumin (Alb-Cys) was increased also in all groups, except D7.

### Discussion:

In this study, we have demonstrated that the binding capacities of the selected ligands are significantly different between cirrhotic patients and patients with no liver dysfunctions. The decreased binding capacities in cirrhotic patients were paralleled by the presence of significantly higher HSA isoforms. This allow us to assume that the most important modifications of albumin structure due to liver dysfunction could be revealed by measuring the unbound fraction of specific ligands spiked in serum. Several studies have reported HSA chemical and/or structural modifications in advanced liver diseases.

Albumin chemical modifications have been extensively reviewed in [7, 10]. Albumin undergoes several post-translational modifications including: acetylation, cysteinylation, homocysteinylation, glutathionylation, glycosylation, glycation, nitrosylation, nitration, phosphorylation and oxidation.

Although oxidation could affect several residues such as methionine, lysine, arginine, and proline, the oxidation of the Cys34 residue is the most studied. This modification was characterized on the basis of the redox state of Cys34 as follows:
1. Human mercaptalbumin (HMA), the reduced and most abundant form of HAS (70 -80% of total HAS in healthy subjects),
2. Nonmercaptalbumin 1 (HNA1), a reversibly oxidized form (20 - 30%) and
3. Nonmercaptalbumin 2 (HNA2) the irreversible oxidized form of albumin (< 5%)[10].

The clinical relevance of these modifications has been recently investigated in advanced liver diseases (1-4,11,12)[1-4, 11, 12]. The significant reductions in HMA percentage with a concomitant increase in HNA1 and HNA2 isoforms have been well documented in end-stage liver injuries. It has also been reported that a progressive increase of the oxidized forms of HSA is detected in cirrhotic patients. In particular, circulating levels of both HNA1 and HNA2 were increased in patients with decompensated cirrhosis and, to a greater extent, in those with acute-on-chronic liver failure, a syndrome characterized by a very high short-term mortality rate [2, 4, 7]. Interestingly, in these patients, HNA2 level significantly correlated with parameters of systemic inflammation and was directly related to disease prognosis. Lately, it was reported that patients with severe alcoholic hepatitis (SAH) had a significant increase in albumin oxidation due to the oxidative stress environment related to the disease. In such conditions, albumin acts as a pro-oxidant and promotes additional oxidative stress and inflammation through activation of neutrophils [13]. Of note, in this study, HNA2 was only increased in SAH and not in chronic alcoholic cirrhotic patients.

Structural alterations involving sites other than Cys34 were also reported. N- or C-terminal truncated, as well as glycated, forms were found in plasma samples from patients with acutely decompensated cirrhosis or severe alcoholic hepatitis [14]. Dimerization of HSA has also been reported in patients with decompensated cirrhosis, although a controversy exists about its pejorative role in the disease. However, the homodimeric isoform with N-terminal truncation was independently associated to disease complications and was able to stratify 1-year survival [14]. Very recently, it has been reported that in SAH patients, excess binding of bilirubin with albumin helps to predict 3-months mortality and that this excessive binding contributes to the observed decrease in binding capacity of dansyl sarcosine to albumin [15].

Therefore, to elaborate the SEB test, we have selected several ligands with known specific binding sites on albumin. The binding sites were chosen in order to cover the most important HSA modifications with reported clinical relevance in liver dysfunctions. On these bases, Au was selected to reveal Cys34 modifications (16-18)[16-18], Cu for its high affinity to the N-terminal site and the multi binding site B [18, 19], L-thyroxine for its 4 binding sites distributed in the 4 cavities of HSA (Tr1 to Tr4) [20], dansylsarconsine for its affinity to the drug site 3 (or diazepam-binding site), which is also the bilirubin binding site [5], and Cd for its high affinity to the multi binding sites A (or Cd binding site) [8].

We observed that serum is able to bind up to 12 atoms of Cu, 150 atoms of Au, 50 atoms of Cd, 2.5 molecules of dansylsarcosine and at least 10 molecules of L-thyroxine per molecule of albumin. These values were much higher than the theoretical and experimental reported ones. For instance, it has been reported that HSA is able to bind less than 2 atoms of Cu [21]. It has been confirmed later that only one specific binding site, namely, the NTS is able to bind Cu, and that the multi metal binding site has a very low affinity for Cu. In this kind of studies, metal binding strategies employing equilibrium dialysis were mostly used [22, 23]. In the later studies, low molecular weight weak chelates were used to prevent metal hydrolysis and subsequent polymerization and thus nonspecific binding. In our experimental conditions in the SEB test, metals hydrolysis could obviously occur which could be responsible for nonspecific bindings due to Van der Waals forces [23]. These nonspecific bindings are even more important when commercial and pure solutions of HSA are incubated with our ligands (supplemental data). The presence of endogenous weak chelators (such as free amino acids) in serum could be the reason behind this. In an in silico model we were able to demonstrate that HSA is able to bind covalently 2 atoms of copper in 2 specific binding sites and up to 40 atoms of copper at different non specific binding sites (Data not shown). Therefore, we decided to apply the SEB test with lower ligands concentrations. All the tested ligands were then able to discriminate cirrhotic patients from non-cirrhotic individuals with 100% sensitivity and 100% specificity in the discovery cohort. The performance of the SEB test was excellent in the development cohort.

The nature and relative abundances of the HSA isoforms found in our analysis are in agreement with previous results [2, 4, 12, 13, 15] and with the results of the SEB test. Indeed, in comparison with patients with normal liver functions, all the cirrhotic patients presented high levels of modified HSA (nitrosylation, cysteinylation and glycation). Nitrosylation and cysteinylation occur on the Cys34 [1], which is consistent with decreased Au binding to HSA in cirrhotic patients. Glycation can occur on Lys199, Lys281, Lys439, and Lys525 [3], all located near the L-thyroxine sites, which might hinder this ligand to bind to HSA. Finally, it has been demonstrated that oxidation of Cys34 could result in a number of conformational changes of HSA [5]. It alters the conformation and dynamics of the entire domain I, as well as of the domain I/II interface, which results in lower binding capacities of endogenous (L-Trp) and exogenous ligands (cefazoline and verapamil), whose binding sites are distant from cys34. This point could explain the decreasing binding capacity of Cd in cirrhotic patients. Cd is reported to coordinate with one His and four carboxylates; however, its location is unknown but should be distant from Cys34 [23].

Despite the very small patient numbers, we observed that the binding capacity of HSA is more decreased in alcohol cirrhotic patients that in those with metabolic cirrhosis or in mixed cirrhosis (Table 1). The HSA-Cys isoform seems to be higher in the first group. The same observation could be done with the results of the Cu SEB test. In addition, patients with the highest Child and MELD scores (patients 2,3 and 8) have the highest HSA-Cys abundances. Patient 19 (not included in the statistics) had a NASH without cirrhosis. The abundance of his HSA-Cys is among the lowest but L-thyroxine and Au binding to HAS was lower than in control patients and higher than in cirrhotic patients. This might be explained by the modifications of Cys34 and L-thyroxine sites and the absence of modification in the NTS, but we have no clue to support this hypothesis so far.

The animal model allowed us to demonstrate that the albumin modifications were mostly acetylation, cysteinylation, glycation and glutathionylation. The SEB test was positive for Cd at Day 14, and for Au and L-thyroxine as soon as D1. Liver injuries after D7 were confirmed by increased serum concentrations of AST and ALT, markers of hepatocyte integrity. As the albumin of rats has not been crystalized and its 3D structure elucidated yet, it is hard to find the link between albumin modifications and binding capacities. However, the results suggest that our test may reveal hepatocyte suffering early, before the current biochemistry biomarkers and that decreased capacity of albumin to bind Cd could be a marker of more advanced liver injuries.

### TABLES:

**Table 1. Patients age and etiology of cirrhosis are indicated along with their Child-Pugh and MELD scores. The abundances of identified albumin isoforms were all higher than in control patients. Isoform percentages are calculated on the basis of the abundance of the native HSA. For all the tested patients the SEB test was positive in comparison to control patients except for Cu in some patients. Ratios are calculated as follows: concentration of ligands in the filtrates (µM)/concentration of albumin (µg/L).**

| **Patient** | **Age** | **Cirrhosis etiology** | **MELD Score** | **Child-Pugh Score** | **HSA Cys (%)** | **HSA Gly (%)** | **HSA NO3 (%)** | **HSA Cys-NO3 (%)** | **Ratio Au/HSA** | **Ratio Cu/HSA** | **Ratio LT/HSA** | **Ratio DS/HSA** |
|---|---|---|---|---|---|---|---|---|---|---|---|---|
| | | ***Reference values in Control patients (mean+*/*-SD)*** | | | ***0.63+*/*-0.11*** | ***0.39+*/*- 0.04*** | ***0.48+*/*-0.02*** | ***0.42+*/*-0.06*** | ***10+*/*-7.9*** | ***4.3+*/*-1.6*** | ***0.09+*/*- 0.03*** | ***0.23+*/*- 0.26*** |
| **1** | 64 | Alcohol | n.a | B | 1.25 | 0.71 | 0.54 | 0.76 | 86.23 | 7.5 | 1.82 | n.a |
| **2** | 74 | | 23 | C14 | 1.16 | 0.57 | 0.57 | 0.73 | 57.8 | 4.97 | 0.86 | n.a |
| **3** | 69 | | 23 | C | 1.84 | 0.81 | 0.63 | 1.21 | 120.09 | 9.33 | 3.04 | n.a |
| **4** | 47 | | 9 | B9 | 0.79 | 0.41 | 0.49 | 0.44 | 102.89 | 15.85 | 1.4 | n.a |
| **5** | 52 | | 15 | C12 | 0.77 | 0.53 | 0.58 | 0.61 | n.a | 5.5 | n.a | n.a |
| **6** | 73 | | 16 | B8 | 1 | 0.45 | 0.47 | 0.6 | 145.77 | 10.45 | 3.33 | 4.77 |
| **7** | 70 | | 14 | C10 | 0.83 | 0.54 | 0.49 | 0.5 | 84.18 | 9.07 | 2.71 | 2.26 |
| **8** | 72 | | 24 | C11 | 1.24 | 0.61 | 0.55 | 0.74 | 89.43 | 6.86 | 2.17 | 5.71 |
| **9** | 80 | NASH | 21 | B9 | 0.9 | 0.53 | 0.53 | 0.64 | n.a | 9.64 | n.a | n.a |
| **10** | 70 | | n.a | n.a | 0.89 | 0.61 | 0.62 | 0.73 | n.a | 7.54 | n.a | n.a |
| **11** | 77 | | n.a | n.a | 0.83 | 0.6 | 0.52 | 0.55 | 61.55 | 6.74 | 2.06 | 7.58 |
| **12** | 77 | | n.a | A | 0.82 | 0.38 | 0.44 | 0.47 | 46.25 | 4.76 | 1.92 | 0.78 |
| **13** | 75 | | 7 | n.a | 0.88 | 0.46 | 0.54 | 0.61 | 64.58 | 5.59 | 1.72 | n.a |
| **14** | 80 | Mixt: alcohol and NASH | 7 | A5 | 0.66 | 0.46 | 0.49 | 0.45 | 97.75 | 8.25 | 2.15 | n.a |
| **15** | 80 | | 29 | B9 | 1.69 | 0.63 | 0.59 | 1.05 | 116.64 | 10.68 | 2.52 | n.a |
| **16** | 78 | | 7 | A6 | 0.73 | 0.39 | 0.47 | 0.45 | 87.56 | 11.02 | 2.55 | 16.38 |
| **17** | 62 | Mixt: alcohol and VHC | 12 | B9 | 0.89 | 0.54 | 0.52 | 0.59 | 39.78 | 5.26 | 1.52 | n.a |
| **18** | 50 | Mixt: VHC, alcohol and NASH | 15 | C | 1.05 | 0.51 | 0.51 | 0.67 | 70.47 | 5.56 | 1.24 | n.a |
| **19** | 60 | NASH without cirrhosis | n.a | n.a | 0.73 | 0.35 | 0.45 | 0.41 | 59.64 | 5.1 | 1.9 | n.a |

**Table 2. Biochemical test results in rats after daily administration of ethanol for different time spans. Group D1 received ethanol for 1 day, D3 for 3 days, D7 for 7 days, D10 for 10 days and D14 for 14 days.**

| **Tests** | **Control rats** | **Group D1** | **Group D3** | **Group D7** | **Group D10** | **Group D14** |
|---|---|---|---|---|---|---|
| **ALB (g/L)** | 14,4 | 13,8 | **12,4*** | **12,2*** | 16,35 | **12,9*** |
| | [12,7-17,6] | [13,2-14,1] | **[11,8-14,2]** | **[11,0-13,8]** | [12,4-17,7] | **[10,2-13,5]** |
| **AST (UI/L)** | 70,8 | 78 | 80 | **96*** | **92*** | **90*** |
| | [61-75] | [70-85] | [52-144] | **[75-157]** | **[81-157]** | **[85-294]** |
| **ALT (UI/L)** | 57 | 63 | 62 | 69 | **78*** | **82*** |
| | [46-61] | [52-71] | [46-123] | [52-97] | **[68-98]** | **[65-104]** |
| **ALP (UI/L)** | 196 | 232 | 180 | 159 | 172 | 230 |
| | [101-325] | [153-304] | [121-329] | [84-263] | [100-222] | [110-327] |
| **Free BILI (µM)** | 0,75 | 0,7 | 0,7 | 0,7 | 0,8 | 0,6 |
| | [0,6-1] | [0,3-1] | [0,5-1,3] | [0,4-4,4] | [0,5-1,1] | [0,4-0,7] |
| **Total BILI (µM)** | 0,75 | 0,8 | 0,8 | 1 | 1 | 0,9 |
| | [0,4-1,3] | [0,4-1,6] | [0,2-1,7] | [0,2-1,9] | [0,8-1,6] | [0,5-1,8] |

### REFERENCES:

Throughout this application, various references describe the state of the art to which this disclosure pertains.
1. Naldi M, Baldassarre M, Domenicali M, et al (2017) Structural and functional integrity of human serum albumin: Analytical approaches and clinical relevance in patients with liver cirrhosis. J Pharm Biomed Anal 144:138-153 . doi: 10.1016/j.jpba.2017.04.023
2. Domenicali M, Baldassarre M, Giannone FA, et al (2014) Posttranscriptional changes of serum albumin: clinical and prognostic significance in hospitalized patients with cirrhosis. Hepatol Baltim Md 60:1851-1860 . doi: 10.1002/hep.27322
3. Naldi M, Baldassarre M, Domenicali M, et al (2016) Mass spectrometry characterization of circulating human serum albumin microheterogeneity in patients with alcoholic hepatitis. J Pharm Biomed Anal 122:141-147 . doi: 10.1016/j.jpba.2016.01.048
4. Oettl K, Birner-Gruenberger R, Spindelboeck W, et al (2013) Oxidative albumin damage in chronic liver failure: relation to albumin binding capacity, liver dysfunction and survival. J Hepatol 59:978-983 . doi: 10.1016/j.jhep.2013.06.013
5. Kawakami A, Kubota K, Yamada N, et al (2006) Identification and characterization of oxidized human serum albumin. A slight structural change impairs its ligand-binding and antioxidant functions. FEBS J 273:3346-3357 . doi: 10.1111/j.1742-4658.2006.05341.x
6. Bar-Or D, Lau E, Winkler JV (2000) A novel assay for cobalt-albumin binding and its potential as a marker for myocardial ischemia-a preliminary report. J Emerg Med 19:311-315
7. Klammt S, Mitzner S, Stange J, et al (2007) Albumin-binding function is reduced in patients with decompensated cirrhosis and correlates inversely with severity of liver disease assessed by model for end-stage liver disease. Eur J Gastroenterol Hepatol 19:257-263 . doi: 10.1097/MEG.0b013e3280101f7d
8. Fanali G, di Masi A, Trezza V, et al (2012) Human serum albumin: from bench to bedside. Mol Aspects Med 33:209-290 . doi: 10.1016/j.mam.2011.12.002
9. El Balkhi S, Poupon J, Trocello J-M, et al (2010) Human plasma copper proteins speciation by size exclusion chromatography coupled to inductively coupled plasma mass spectrometry. Solutions for columns calibration by sulfur detection. Anal Chem 82:6904-6910 . doi: 10.1021/ac101128x
10. Bonneau E, Tétreault N, Robitaille R, et al (2016) Metabolomics: Perspectives on potential biomarkers in organ transplantation and immunosuppressant toxicity. Clin Biochem 49:377-384 . doi: 10.1016/j.clinbiochem.2016.01.006
11. Spinella R, Sawhney R, Jalan R (2016) Albumin in chronic liver disease: structure, functions and therapeutic implications. Hepatol Int 10:124-132 . doi: 10.1007/s12072-015-9665-6
12. Stauber RE, Spindelboeck W, Haas J, et al (2014) Human nonmercaptalbumin-2: a novel prognostic marker in chronic liver failure. Ther Apher Dial Off Peer-Rev J Int Soc Apher Jpn Soc Apher Jpn Soc Dial Ther 18:74-78 . doi: 10.1111/1744-9987.12024
13. Das S, Maras JS, Hussain MS, et al (2017) Hyperoxidized albumin modulates neutrophils to induce oxidative stress and inflammation in severe alcoholic hepatitis. Hepatol Baltim Md 65:631-646 . doi: 10.1002/hep.28897
14. Baldassarre M, Domenicali M, Naldi M, et al (2016) Albumin Homodimers in Patients with Cirrhosis: Clinical and Prognostic Relevance of a Novel Identified Structural Alteration of the Molecule. Sci Rep 6:35987 . doi: 10.1038/srep35987
15. Das S, Maras JS, Maiwall R, et al (2018) Molecular Ellipticity of Circulating Albumin-Bilirubin Complex Associates With Mortality in Patients With Severe Alcoholic Hepatitis. Clin Gastroenterol Hepatol Off Clin Pract J Am Gastroenterol Assoc 16:1322-1332.e4 . doi: 10.1016/j.cgh.2017.11.022
16. Li Y, Yan X-P, Chen C, et al (2007) Human serum albumin-mercurial species interactions. J Proteome Res 6:2277-2286 . doi: 10.1021/pr0700403
17. Shen X-C, Liang H, Guo J-H, et al (2003) Studies on the interaction between Ag(+) and human serum albumin. J Inorg Biochem 95:124-130
18. Sokolowska M, Wszelaka-Rylik M, Poznański J, Bal W (2009) Spectroscopic and thermodynamic determination of three distinct binding sites for Co(II) ions in human serum albumin. J Inorg Biochem 103:1005-1013 . doi: 10.1016/j.jinorgbio.2009.04.011
19. Peters T (1995) Ligand Binding by Albumin. In: All About Albumin. Elsevier, pp 76-132
20. Petitpas I, Petersen CE, Ha C-E, et al (2003) Structural basis of albumin-thyroxine interactions and familial dysalbuminemic hyperthyroxinemia. Proc Natl Acad Sci U S A 100:6440-6445 . doi: 10.1073/pnas.1137188100
21. Appleton DW, Sarkar B (1971) The Absence of Specific Copper(II)-binding Site in Dog Albumin A COMPARATIVE STUDY OF HUMAN AND DOG ALBUMINS. J Biol Chem 246:5040-5046
22. Masuoka J, Saltman P (1994) Zinc(II) and copper(II) binding to serum albumin. A comparative study of dog, bovine, and human albumin. J Biol Chem 269:25557-25561
23. Sendzik M, Pushie MJ, Stefaniak E, Haas KL (2017) Structure and Affinity of Cu(I) Bound to Human Serum Albumin. Inorg Chem 56:15057-15065. doi: 10.1021/acs.inorgchem.7b02397

## Claims

1. A method for determining whether a subject suffers from a liver dysfunction comprising measuring the binding capacities of gold (Au), copper (Cu), cadmium (cd), L-thyroxine and dansylsarcosine to serum albumin in a sample that has been obtained from the subject wherein said measured binding capacities indicate whether the subject suffers from a liver dysfunction.

2. The method of claim 1 wherein the binding capacities of gold (Au), copper (Cu), cadmium (cd), L-thyroxine and dansylsarcosine are compared with their predetermined reference values, and the detection of a difference between the binding capacities of gold (Au), copper (Cu), cadmium (cd), L-thyroxine and dansylsarcosine and the predetermined reference values indicates if the subject suffers from a liver dysfunction.

3. The method of claim 1 comprising the steps of i) providing a serum sample that has been obtained from the subject, ii) exposing the serum sample to a predetermined amount of gold (Au), copper (Cu), cadmium (cd), L-thyroxine and dansylsarcosine for a time sufficient for allowing the serum albumin to bind to said gold (Au), copper (Cu), cadmium (cd), L-thyroxine and dansylsarcosine, iii) measuring the amount of the free gold (Au), copper (Cu), cadmium (cd), L-thyroxine and dansylsarcosine in the serum sample, and iv) optionally calculating a ratio between the free amount and the concentration of the serum albumin.

4. The method of claim 1 wherein the binding capacities are determined by mass spectrometry, in particular by inductively coupled plasma mass spectrometry (ICP-MS).

5. The method of claim 3 wherein a score which is a composite of the measured binding capacities is determined and compared to a reference value wherein a difference between said score and said reference value indicates whether the subject suffers from a liver dysfunction.

6. The method of claim 3 which comprises the use of a classification algorithm.

7. The method of claim 6 comprising a) measuring the binding capacities of gold (Au), copper (Cu), cadmium (cd), L-thyroxine and dansylsarcosine b) implementing a classification algorithm on data comprising the measured binding capacities so as to obtain an algorithm output; c) determining the probability that the subject suffers from a liver dysfunction.

## Patentansprüche

1. Verfahren zum Bestimmen, ob ein Proband an einer Leberfunktionsstörung leidet, umfassend das Messen der Bindungskapazitäten von Gold (Au), Kupfer (Cu), Cadmium (Cd), L-Thyroxin und Dansylsarcosin an Serumalbumin in einer Probe, die von dem Probanden erhalten wurde, wobei die gemessenen Bindungskapazitäten anzeigen, ob der Proband an einer Leberfunktionsstörung leidet.

2. Verfahren nach Anspruch 1, wobei die Bindungskapazitäten von Gold (Au), Kupfer (Cu), Cadmium (Cd), L-Thyroxin und Dansylsarcosin mit ihren vorbestimmten Referenzwerten verglichen werden, und das Erkennen einer Differenz zwischen den Bindungskapazitäten von Gold (Au), Kupfer (Cu), Cadmium (Cd), L-Thyroxin und Dansylsarcosin und den vorbestimmten Referenzwerten anzeigt, ob der Proband an einer Leberfunktionsstörung leidet.

3. Verfahren nach Anspruch 1, umfassend die Schritte des i) Bereitstellens einer Serumprobe, die von dem Probanden erhalten wurde, ii) Aussetzens der Serumprobe einer vorbestimmten Menge an Gold (Au), Kupfer (Cu), Cadmium (Cd), L-Thyroxin und Dansylsarcosin für eine Zeit, die ausreicht, um das Serumalbumin an das Gold (Au), Kupfer (Cu), Cadmium (Cd), L-Thyroxin und Dansylsarcosin zu binden, iii) Messens der Menge des freien Goldes (Au), Kupfers (Cu), Cadmiums (Cd), L-Thyroxins und Dansylsarcosins in der Serumprobe und iv) optional Berechnens eines Verhältnisses zwischen der freien Menge und der Konzentration des Serumalbumins.

4. Verfahren nach Anspruch 1, wobei die Bindungskapazitäten durch Massenspektrometrie, insbesondere durch induktiv gekoppelte Plasmamassenspektrometrie (ICP-MS) bestimmt werden.

5. Verfahren nach Anspruch 3, wobei eine Punktzahl, die sich aus den gemessenen Bindungskapazitäten zusammensetzt, bestimmt und mit einem Referenzwert verglichen wird, wobei eine Differenz zwischen der Punktzahl und dem Referenzwert anzeigt, ob der Proband an einer Leberfunktionsstörung leidet.

6. Verfahren nach Anspruch 3, das die Verwendung eines Klassifizierungsalgorithmus umfasst.

7. Verfahren nach Anspruch 6, umfassend a) Messen der Bindungskapazitäten von Gold (Au), Kupfer (Cu), Cadmium (Cd), L-Thyroxin und Dansylsarcosin; b) Implementieren eines Klassifizierungsalgorithmus auf Daten, die die gemessenen Bindungskapazitäten umfassen, sodass eine Algorithmusausgabe erhalten wird; c) Bestimmen der Wahrscheinlichkeit, dass der Proband an einer Leberfunktionsstörung leidet.

## Revendications

1. Procédé permettant de déterminer si un sujet souffre d'un dysfonctionnement hépatique, comprenant la mesure des capacités de liaison de l'or (Au), du cuivre (Cu), du cadmium (Cd), de la L-thyroxine et de la dansylsarcosine à l'albumine sérique dans un échantillon qui a été obtenu à partir du sujet, dans lequel lesdites capacités de liaison mesurées indiquent si le sujet souffre d'un dysfonctionnement hépatique.

2. Procédé selon la revendication 1, dans lequel les capacités de liaison de l'or (Au), du cuivre (Cu), du cadmium (Cd), de la L-thyroxine et de la dansylsarcosine sont comparées à leurs valeurs de référence prédéterminées, et la détection d'une différence entre les capacités de liaison de l'or (Au), du cuivre (Cu), du cadmium (Cd), de la L-thyroxine et de la dansylsarcosine et les valeurs de référence prédéterminées indique si le sujet souffre d'un dysfonctionnement hépatique.

3. Procédé selon la revendication 1, comprenant les étapes de i) fourniture d'un échantillon de sérum qui a été obtenu à partir du sujet, ii) exposition de l'échantillon de sérum à une quantité prédéterminée d'or (Au), de cuivre (Cu), de cadmium (Cd), de L-thyroxine et de dansylsarcosine pendant une durée suffisante pour permettre à l'albumine sérique de se lier auxdits or (Au), cuivre (Cu), cadmium (Cd), L-thyroxine et dansylsarcosine, iii) mesure de la quantité d'or (Au), de cuivre (Cu), de cadmium (Cd), de L-thyroxine et de dansylsarcosine libres dans l'échantillon de sérum, et iv) calcul facultatif d'un rapport entre la quantité libre et la concentration de l'albumine sérique.

4. Procédé selon la revendication 1, dans lequel les capacités de liaison sont déterminées par spectrométrie de masse, en particulier par spectrométrie de masse avec plasma à couplage inductif (ICP-MS).

5. Procédé selon la revendication 3, dans lequel un score qui est une combinaison des capacités de liaison mesurées est déterminé et comparé à une valeur de référence, dans lequel une différence entre ledit score et ladite valeur de référence indique si le sujet souffre d'un dysfonctionnement hépatique.

6. Procédé selon la revendication 3, qui comprend l'utilisation d'un algorithme de classification.

7. Procédé selon la revendication 6, comprenant a) la mesure des capacités de liaison de l'or (Au), du cuivre (Cu), du cadmium (Cd), de la L-thyroxine et de la dansylsarcosine ; b) la mise en oeuvre d'un algorithme de classification sur des données comprenant les capacités de liaison mesurées de manière à obtenir une sortie d'algorithme ; c) la détermination de la probabilité que le sujet souffre d'un dysfonctionnement hépatique.
